# EUROPEAN PATENT APPLICATION

(11) **EP 4 498 386 A1**
(43) Date of publication of application: **29.01.2025**
(21) Application number: 23188467.7
(22) Date of filing: 28.07.2023
(51) Int. Cl.: G16H 40/67, G16H 80/00

(54) **COMMUNICATION SYSTEM ADJUSTED USING PHYSIOLOGICAL DATA**

(71) Applicant: Koninklijke Philips N.V., 5656 AG Eindhoven (NL)
(72) Inventor: WESTERINK, Joanne Henriëtte Desirée Monique, Eindhoven (NL); HART DE RUIJTER-BEKKER, Evelijne Machteld, Eindhoven (NL); LACROIX, Joyca Petra Wilma, 5656AG Eindhoven (NL); VAN EE, Raymond, Eindhoven (NL); KLAMING, Laura, Eindhoven (NL); MENA BENITO, Maria Estrella, Eindhoven (NL); RUTJES, Heleen, Eindhoven (NL); JELFS, Sam Martin, Eindhoven (NL); MENDOZA, Jose Luis Diaz, Eindhoven (NL); ANAND, Shreya, Eindhoven (NL)
(74) Representative: Philips Intellectual Property & Standards

(57) **Abstract**

Disclosed herein is a medical system (100, 300) comprising a communication system (110) configured for establishing a communication session between multiple communication devices (104). The medical system further comprises a memory (114) storing machine executable instructions (120). The medical system further comprises a computational system, wherein execution of the machine executable instructions causes the computational system to repeatedly: receive (200) user specific physiological data (122) from the multiple communication devices using the communication system; calculate (202) a user specific control signal (124) for the multiple telecommunication devices at least partially using the user specific physiological data; and control (204) the communication session using the user specific control signal.

## Description

### TECHNICAL FIELD

The invention relates to medical communications and telemedicine.

### BACKGROUND

Medical communications may involve a number or participants each sharing audio and or visual data from different sources. For example, participants may share medical images to discuss cases. In other examples participants may participate in the examination or diagnosis of a clinical subject. In other examples participants may receive training on how to use a medical imaging system. Images of the medical imaging system, as well as user interfaces may be reproduced for participants.

### SUMMARY

The invention provides for a medical system, a computer program, and a method in the independent claims. Embodiments are given in the dependent claims.

In one aspect the invention provides for a medical system that comprises a communication system configured for establishing a communication session between multiple communication devices. The medical system further comprises a memory storing machine-executable instructions. The medical system further comprises a computational system. Execution of the machine-executable instructions causes the computational system to repeatedly receive user-specific physiological data from the multiple communication devices using the communication system. Execution of the machine-executable instructions further causes the computational system to repeatedly calculate a user-specific control signal for the multiple telecommunication devices at least partially using the user-specific physiological data. Execution of the machine-executable instructions further causes the computational system to repeatedly control the communication session using the user-specific control signal.

In another aspect the invention provides for a method of operating a medical system. The medical system comprises a communication system that is configured for establishing a communication session between multiple communication devices. The method comprises repeatedly receiving user-specific physiological data from the multiple communication devices using the communication system. The method further comprises repeatedly calculating a user-specific control signal for the multiple telecommunication devices at least partially using the user-specific physiological data. The method further comprises repeatedly controlling the communication session using the user-specific control signal.

In another aspect the invention provides for a computer program that comprises machine-executable instructions for execution by a computational system that is controlling a communication system. Execution of the machine-executable instructions causes the computational system to repeatedly receive user-specific physiological data from the multiple communication devices using the communication system. Execution of the machine-executable instructions further causes the computational system to repeatedly calculate a user-specific control signal for the multiple telecommunication devices at least partially using the user-specific physiological data. Execution of the machine-executable instructions further causes the computational system to repeatedly control the communication session using the user-specific control signal.

### BRIEF DESCRIPTION OF THE DRAWINGS

In the following preferred embodiments of the invention will be described, by way of example only, and with reference to the drawings in which:
Fig. 1 illustrates an example of a medical system.
Fig. 2 shows a flow chart which illustrates a method of using the medical system of Fig. 1.
Fig. 3 illustrates an example of a medical system.
Fig. 4 shows a flow chart which illustrates a method of using the medical system of Fig. 3.
Fig. 5 shows a flow chart which illustrates a further method of operating a medical system as is depicted in Fig. 1 and 3.
Fig. 6 illustrates a group of visual data which comprises two categories.
Fig. 7 illustrates an example of a private warning.

### DESCRIPTION OF EMBODIMENTS

Like numbered elements in these Figures are either equivalent elements or perform the same function. Elements which have been discussed previously will not necessarily be discussed in later Figures if the function is equivalent.

Examples may have the benefit that the physiological data from the multiple communication devices is used to generate a user-specific control signal. This user-specific control signal may be used to control the communication session and tailor its operation to the different users using the different multiple communication devices. This may have the benefit that the communication session may be personalized or optimized for different users. The use of physiological data enables this personalization to be performed in a way based on the user-specific physiological data. The user-specific physiological data as used herein may encompass measurements or sensor data which are descriptive of physiological processes or measurements made on a specific user. These measurements are therefore based on the physical state of the user or on involuntary reactions. Examples may form a closed control loop that may be used to optimize the user's experience based on this user-specific physiological data in a closed control loop.

In another example the communication session is a teleconference session. For example, the user-specific control signal may be used to control or adjust the behavior or operation of the teleconference session.

In another example the multiple communication devices are telecommunication devices. For example, the multiple telecommunication devices could be a smartphone, a computer system, a teleconferencing system, a tablet computer, a portable telecommunication device and combinations thereof.

In another example the memory further stores at least one group of visual data. The at least one group of visual data is divided into multiple categories. Each of the at least one group of visual data may for example represent a presentation or information to be presented using the communication system. The multiple categories into which the at least one group of visual data is divided may represent differing amounts of information which may be presented using the communication system. Execution of the machine-executable instructions further causes the computational system to repeatedly use the user-specific control signal to choose at least one category of the multiple categories. Execution of the machine-executable instructions further causes the computational system to repeatedly construct a user-specific rendering of the at least one group of visual data using the at least one category selected using the user-specific control signal.

Execution of the machine-executable instructions further causes the computational system to repeatedly transmit the user-specific rendering to the multiple communication devices. In this example the choice of the multiple categories affects the user-specific rendering. Depending on the user-specific control signal the rendering may be adjusted to a particular user. This for example may be used to increase or decrease the amount of visual data which is presented to a user. This may be useful in adjusting the amount of information which is presented to a user in a way such that the user will gain the greatest benefit from participating in the communication session.

In another aspect execution of the machine-executable instructions further causes the computational system to provide a public warning signal or a private warning signal if the user-specific control signal is within a predetermined range. The user-specific control signal could for example be used to indicate when a participant is not paying attention or if stress levels, such as may be measured using the user-specific physiological data, are within a range such that participation by the subject in a communication session is sub-optimal. A public warning may for example be broadcast to one or more participants in the communication session to warn them that a participant or particular participant has the specific control signal within the predetermined range. The private warning signal could for example be presented to the actual participant for which the user-specific control signal is within the predetermined range or to a presenter. This may be useful feedback to both individuals either to change the content of the communication session, to make it more interesting, or as a reminder to pay attention.

The medical system could give a private warning signal to any member of staff with high (or increasing) fatigue/stress levels, as possibly indicated by the user specific physiological data, over an extended period of time. It could advise a specific user to consider whether adapting working patterns, or working hours, or private life scheduling will help to alleviate the fatigue/stress. For this an additional app on a smartphone or running on one of the multiple communication devices could be made available to help minimize and manage fatigue/stress levels. The app could allow them to do any one of the following:
- Closely and timely monitor how tired/stressed they are at specific moments in time
- Identify from a large set of potential sources which contribute strongest to feelings of fatigue or stress
- Closely and timely monitor their bodily warning signals and know how these should be interpreted
- Determine when behavioral changes are needed, and which behavioral changes may improve energy levels and relaxation
- Learn how to cope with persistent feelings of fatigue/stress
- Predict levels of fatigue/stress based on personal factors (e.g., activities, sleep quality, cognitive load) and take preventive measures.

In some embodiments the public warning signal and/or the private warning signal could for example be a popup window or user interface that warns the presenter and/or participant if they are beginning to lose attention. This for example may be useful in situations where the communication is for discussing a particular medical case or in a remote telemedicine procedure. For example, if a remote physician was not paying proper attention this may have a detrimental effect on the subject being examined or discussed.

In another example execution of the machine-executable instructions further causes the computational system to iterate through modifications of the private warning signal if the user-specific control signal is within the predetermined range for over a predetermined time interval. For example, if the private warning signal has been presented and there is no change in the user-specific control signal then modification of the private warning signal could be used to try to gain the attention of the user in an attempt to be able to modify the user-specific control signal.

In another example execution of the machine-executable instructions further causes the computational system to calculate a composite control signal using the user-specific score received from multiple telecommunication devices and then schedules a future communication session using the composite control signal. The composite control signal may for example be an average or weighted average of the user-specific scores received from the various multiple telecommunication devices. Depending upon this composite control signal this can be used to schedule an optimal time for a subsequent communication session.

In another example the user-specific physiological data comprises sensor data collected by the multiple communication devices. This may be advantageous because the physiological data can be linked directly to a particular user. In some examples the sensor data is from user-specific wearable devices. For example, a user may have a smartwatch or other sensor that is connected to a particular communication device. The sensor data could for example include such things as heart rate, skin conductivity or other quantities such as blood sugar.

In another embodiment execution of the machine-executable instructions further causes the computational system to repeatedly receive user-specific communication data from the multiple communication devices. The user-specific control signal is at least partially calculated using the user-specific communication data. The user-specific communication data as used herein may encompass data which is received from a communication device during the course of communication between different users which are participating in a communication session. This for example may include such things as text data, image or video streams, and also potentially audio data. For example, there may be audio streams of particular users speaking or asking questions.

In another embodiment the user-specific control signal is calculated using the correlation between the user-specific communication data and the user-specific physiological data. This may be performed in several different ways. In some cases there may be a correlation calculated directly between the data that makes up the user-specific communication data and the data which makes up the user-specific physiological data.

For example, various measurements in the user-specific communication data could be compared or correlated to data in the user-specific physiological data. In some instances the user-specific communication data and/or the user-specific physiological data may involve several measurements for each one. In which case for example, as an intermediate step, there could be a communication score which is calculated using the user-specific communication data and/or a physiological score calculated for the user-specific physiological data.

The correlation between these two scores could then be calculated. This example may be advantageous because it may enable a means of comparing the communication score with a physiological score of the subject. For example, it could be able to determine baselines for both values and/or determine how the physiological data affects the ability of a participant to communicate.

In another example the communication data comprises a user-specific audio stream collected by multiple telecommunication devices. The memory further comprises a speech recognition module that is configured to output one or more speech metrics in response to receiving the user-specific audio stream. Execution of the machine-executable instructions further causes the computational system to receive the one or more speech metric in response to inputting the user-specific audio stream into the speech recognition module. Execution of the machine-executable instructions further causes the computational system to at least partially calculate the user-specific control signal using the one or more speech metric.

The speech recognition module could for example be a commercially available speech recognition module or it may be a neural network such as a convolutional neural network or neural network constructed of fully connected layers which is able to analyze segments or whole streams of audio. This embodiment may be beneficial because it may provide for a means of quantifying data from the speech received from a particular user. This may be particularly successful in determining how successfully a user is participating in the communication session or if there is a subtle change in the user's behavior which may impair that user's participation effectively in the communication session.

In another embodiment the one or more speech metric comprises a measured reaction time to a question. For example, the delay that a user has in response to an asked question may be a useful metric. In another example the one or more speech metric comprises a pitch range or change in pitch range of the subject's speech in the audio stream. The change in the subject's use of pitch may also indicate a change in the subject's comprehension or attention to the communication session.

The one or more speech metric further comprises a volume range or change in the volume range of the subject's speech. This may also be useful in determining if there are changes in the subject's participation in the communication session.

The one or more speech metric further comprises an emotional state estimated using natural language processing and/or sentiment analysis.

In another embodiment the one or more speech metric comprises a syntactic analysis comparing the audio stream of the multiple communication devices to detect repeated questions or interruptions of speech. This for example, may be beneficial when trying to determine if a subject comprehends or is having difficulty understanding a particular topic being discussed in the communication session. The syntactic analysis may for example be performed using a large language model.

A large language model (LLM) as used herein encompasses a neural network architecture, typically built up of transformers (encoders and decoders) with self-attention layers and residual connections, that is a language model that has been trained using unlabeled text using selfsupervised or semi-supervised learning. Typically, LLMs are trained using billions of words. LLMs may for example be trained in an autoregressive form where given a segment of text the model predicts the next word (token) or words (tokens). Another mode of training is where words or tokens within a sentence are missing and the LLM predicts the missing words or tokens. Both types of LLMs may be configured to be used in the so-called prompting paradigm where a text query or statement is input into the LLM and the LLM outputs a completion or statement. The LLMs described herein are configured to operate in the prompting paradigm. Example LLMs are GPT-3, GPT-4, BERT, LLaMA, and others. The LLM may be trained for specific tasks using reinforcement learning or by reinforcement learning from human feedback (RLHF). The output of a preexisting LLM may be adjusted using fine-tuning. In fine-tuning a new set of weights connecting the final layer of the language model may be trained with specific data. Typically, this is done by freezing the other weights (other than the final output layer) in the neural network so that only the final output and format is affected.

In this example the large language model is a large language model that has been trained or configured (using fine tuning) to generate a score which indicates how often speech is interrupted or how often questions are repeated in response to receiving text generated from the user specific audio stream.

In another example the user-specific communication data comprises a video stream that comprises user-specific images collected by the multiple communication devices. This for example may be individual still images or it may be video received from the multiple communication devices as would be received during a teleconferencing session. The memory further comprises an image recognition module configured to output one or more image metrics in response to receiving the video stream. The image recognition module may for example be a commercial image recognition module to determine the one or more metrics or it may be a custom image recognition module. For example, a convolutional neural network may be useful for identifying various gestures or changes in videos or individual images within a video stream.

Execution of the machine-executable instructions further causes the computational system to receive the one or more image metric in response to inputting the image stream into the image recognition module. Execution of the machine-executable instructions further causes the computational system to at least partially calculate the user-specific control signal using the one or more image metric. This example may be beneficial because it may provide a means of adjusting the communication session using readily available video data.

In another example the one or more image metric comprises a facial score assigned using facial expression analysis.

In another embodiment the one or more image metric comprises a gesture score assigned using a gesture analysis and/or frequency of gestures using gesture analysis. For example, a neural network or other software module may be used to identify the positions of joints or limbs of the subject and these could be a mapped to a predetermined directory or mapping of gestures.

In another embodiment the one or more image metric comprises a gaze analysis scoring correlated to time viewing the device specific rendering. For example, this may be used for directly detecting if the user is actually looking at the device specific rendering or not.

In another embodiment the one or more image metric may comprise a heart rate. In particular a color camera may be used to notice the change in the skin of a subject and detect the heart rate directly.

In another embodiment the one or more image metric comprises a breathing rate. For example, the breathing rate may be detected by noticing the motion of a subject's chest.

In another example, the user-specific communication data further comprises text metadata. The user-specific control signal is at least partially calculated using the text metadata. The text metadata comprises any one of the following collected by the multiple communication devices: a user-specific reaction time to a received message, a user-specific typing speed, a user-specific questionnaire response, and user-specific small talk response data received in response to a chatbot conversation data. This example may be beneficial because it may provide for an objective means of measuring the communication of a particular user by examining the text metadata.

In another example execution of the machine-executable instructions further causes the computational system to receive a user-specific baseline control signal. These are specific control signals adjusted using the user-specific baseline control signal. For example, individual users may have variations in what their normal user-specific control signal is. The user-specific baseline control signal may be a means of adjusting or fine tuning the response of the communication system for particular users.

In another example execution of the machine-executable instructions further causes the computational system to repeatedly archive the user-specific control signal received from the multiple communication devices and then adjust the user-specific control signal using the repeatedly archived user-specific control signal. In this example, the specific control signal is archived and then used to adjust the user-specific control signal later. This may be beneficial in adjusting the behavior of the communication system for particular users.

In another example execution of the machine-executable instructions further causes the computational system to receive subject data descriptive of a clinical subject. The subject data could be data that is descriptive of a current clinical subject that is present in the communication system or historical data. For example, the subject data could be subject data which is descriptive of a subject which is being examined in a telemedicine session. The user-specific control signal is at least partially calculated using the subject data. This example may be beneficial because it may provide for a means of adjusting the behavior of the communication system based on the presence or the clinical status of a subject that is present. This for example may be useful in adjusting individual user-specific control signals because the presence of a subject may affect the communication of individual users of the medical system. For example, it may be advantageous to ensure that the communication of the participants is adequate when there is a clinical subject present.

In another embodiment execution of the machine-executable instructions further causes the computational system to adjust the user-specific control signal using a correlation between the user-specific communication data and the subject data. This embodiment may be beneficial because it may be able to note the effect on the user-specific communication data for a particular user when there is a subject present. The subject data may for example include such things as subject outcome or the status of a subject at a particular time after the communication session. This may be useful in ensuring that the communication system is functioning in a way such that the subject outcome is improved or attains a specific level.

In another example execution of the machine-executable instructions further causes the computational system to receive an indication of a current presenter. Execution of the machine-executable instructions further causes the computational system to adjust the user-specific control signal using a user-specific modifier determined using the indication of the current presenter. This may be beneficial because it may be used to adjust the effect of a particular presenter on the communication of the individual users using the multiple communication devices.

In another example the medical system further comprises a medical imaging system. The at least one group of visual data comprises acquired medical images and/or a control user interface rendering. This embodiment may be beneficial because the visual data which is displayed from the medical imaging system can be adjusted for a particular user.

In another embodiment the medical system further comprises a medical image database. The at least one group of visual data at least partially comprises data retrieved from the medical imaging database. Likewise, the data which is displayed from the medical image database can be adjusted for a particular user. This may be particularly advantageous as to not provide any superfluous or unneeded data which may distract the subject.

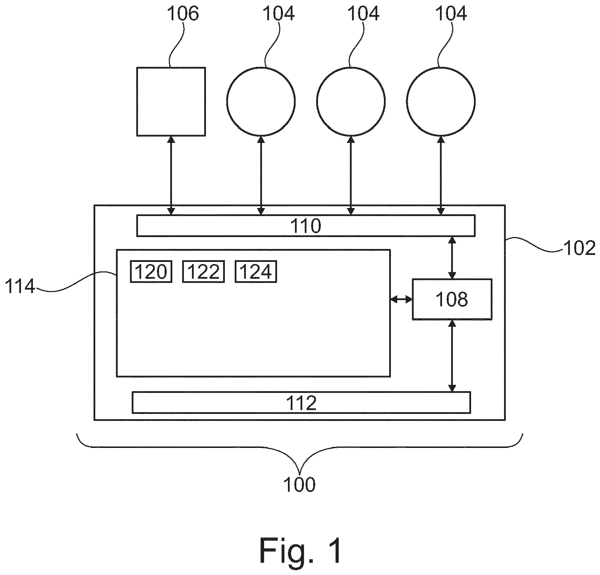

Fig. 1 illustrates an example of a medical system 100. The medical system 100 is shown as comprising a computer 102 and a number of communication devices 104. The computer 102 may also function as a communication device. The medical system 100 is also shown as optionally comprising a medical image source 106 which may for example be access to a medical image database or to a medical imaging system such as a magnetic resonance imaging system, computed tomography system, a digital fluoroscope, a digital X-ray system, or a positron emission tomography system. The computer 102 is shown as comprising a computational system 108. The computational system 108 is shown as being in communication with a network interface 110. In this particular example the network interface 110 may be used as the basis of a communication system between the computer 102 and the various communication devices 104. The computational system 108 is shown as being in further communication with a user interface 112. The user interface 112 may for example provide a text, video, and/or audio interface for the user of the computer 102.

The computational system 108 is further shown as being in communication with a memory 114. The memory is shown as containing machine-executable instructions 120. The machine-executable instructions 120 enable the computational system 108 to perform various text, visual, and data manipulations and also to control the function of the medical system 100. The memory 114 is further shown as containing user-specific physiological data 122. The user-specific physiological data 122 may be physiological data descriptive of a particular user and acquired by one or more of the communication devices 104. For example, a user of one of the communication devices 104 may wear a smartwatch which acquires and transfers the physiological data to the communication device 104, which then sends it via the network interface 110 to the computational system 108. The memory 114 is further shown as containing a user-specific control signal 124 that was calculated using user-specific physiological data 122. The user-specific control signal 124 may be calculated for the individual communication devices 104 specifically to modify the operation of the medical system 100 for the benefit of a particular user.

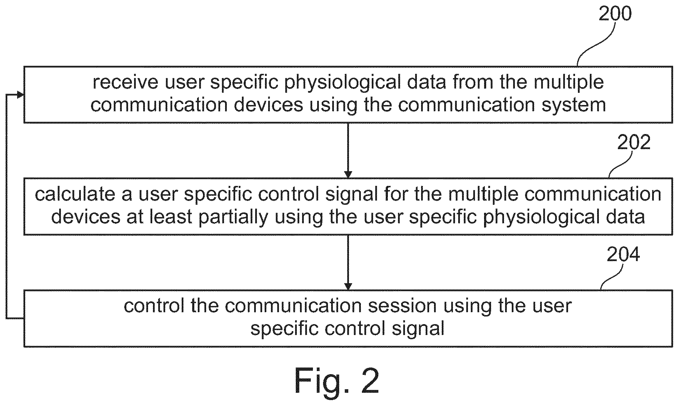

Fig. 2 shows a flowchart which illustrates a method of operating the medical system 100 of Fig. 1. In step 200 user-specific physiological data 122 is received from the multiple communication devices 104 using the communication system 110. In step 202, the user-specific control signal 124 is calculated for the multiple communication devices 104 at least partially using the user-specific physiological data 122. In step 204, the communication session is controlled using the user-specific control signal 124 to adjust the communication system or the experience for each user of the individual devices 104. Fig. 2 forms a closed control loop. As the communication session is adjusted the user-specific physiological data may change. The behavior of the communication session may then be adjusted on the basis of the user-specific physiological data and may adapt over time and be used to improve the quality of the communication session.

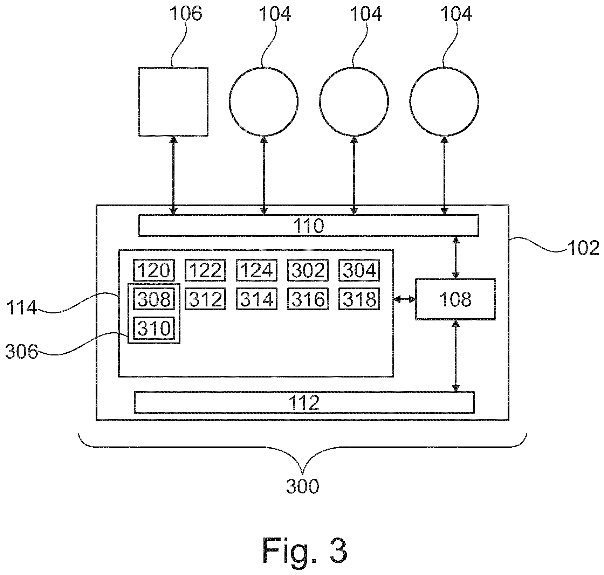

Fig. 3 illustrates a further example of a medical system 300. The medical system 300 depicted in Fig. 3 is similar to the medical system 100 in Fig. 1. The memory 114 is shown as containing additional data.

The memory 114 is further shown as containing user-specific communication data 302 that was collected from the different communication devices 104 for specific users. The memory 114 is further shown as containing subject data 304, which may be descriptive of historical data of a clinical subject, data descriptive of a clinical subject participating in the communication session or data descriptive of data provided by a medical imaging system or medical imaging database. The memory 114 is further shown as containing visual data 306 that is divided into several categories 308, 310. The idea behind having the different categories 308, 310 is that not all of the visual data 306 may be relevant to all participants in the communication session. In some instances, if the particular user is having a difficult time understanding the data being presented or from the user-specific physiological data it is indicated that the subject may be under stress, there may be a benefit in increasing or decreasing the amount of visual data which is displayed. The visual data can be divided into a number of different categories. In this example there are two to represent two levels of data which may be presented to a particular user.

The memory 114 is further shown as containing a user-specific rendering 312 which would be generated using either the first category visual data 308 or the second category visual data 310 depending upon the specific control signal 124. The memory 114 is also shown as containing a public or private warning signal 314 which could for example be rendered on a user interface. The memory 114 is further shown as containing a composite control signal 316 that was calculated from multiple user-specific control signals 124 for multiple communication devices 104. This composite control signal 316 may be indicative of the overall condition or level of communication in the communication system for multiple participants. The composite control signal 316 can then be used to schedule a future communication system. The memory 114 is shown as containing an actual schedule for future communication session 318 that was created using at least partially the composite control signal 316.

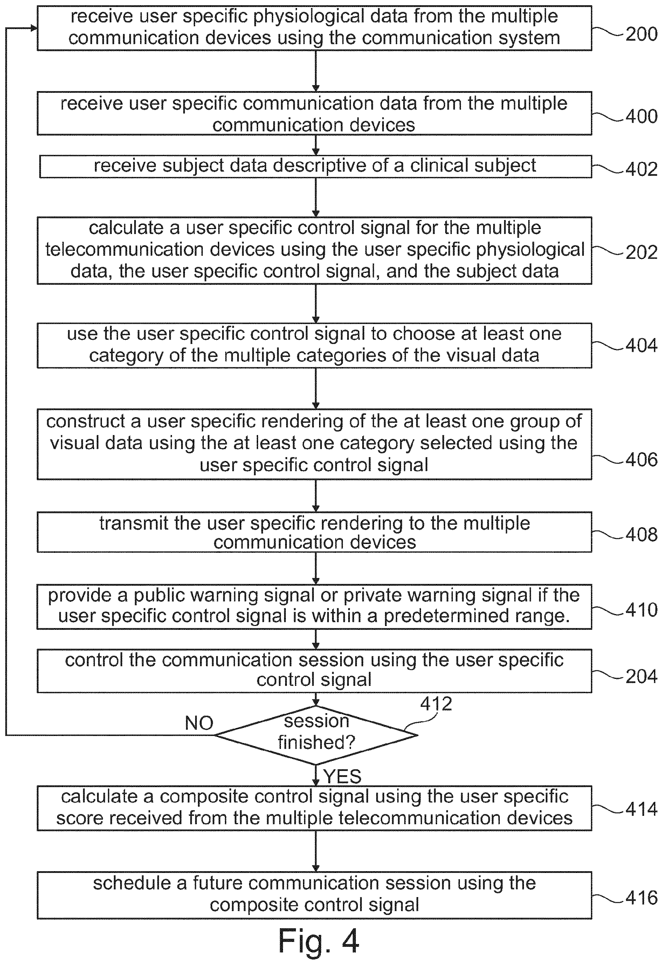

Fig. 4 shows a flowchart which illustrates a method of operating the medical system 300 of Fig. 3. The method illustrated in Fig. 4 is similar to the method illustrated in Fig. 2. Step 200 is performed as was illustrated in Fig. 2. In step 400 the user-specific communication data 302 is received from the multiple communication devices 104. The user-specific communication data 302 may take different forms in different examples. It could for example represent any sort of information which is exchanged during the course of the communication session. It may therefore comprise text, audio, and/or visual or video data. In step 402 subject data descriptive of a clinical subject is received. As is mentioned above, the subject data may be descriptive of historical data or the data descriptive of a clinical subject who is present or is the subject of the communication session.

In step 202 the user-specific control signal 124 is calculated as was mentioned before in Fig. 1, except that it is additionally calculated using the user-specific physiological data 122, the user-specific communication data 302, and the subject data 304. In step 404, the user-specific control signal 124 is used to choose at least one category 308, 310 of the visual data 306. In step 406, the user-specific rendering 312 is constructed using the at least one group of visual data 308, 310 that was selected. In step 408, the user-specific rendering 312 is transmitted to the multiple communication devices. This is a user-specific rendering and was constructed for a particular user using a particular device 104. In step 410, the public or private warning signal 314 is provided if the user-specific control signal 124 is within a predetermined range. This may for example cause the private or public warning signal 314 to be displayed on multiple displays of the devices 104 or for a particular user or for a presenter.

Step 204 is performed as was described in Fig. 2. Step 412 is a decision box, the question is: is the session finished. If the answer is "no" the method repeats to block 200 and the closed control loop is continued. If the session is finished the method proceeds to block 414. In block 414 a composite control signal 316 is calculated using the user-specific score received from the multiple telecommunication devices 104. This may for example be an average or weighted average of values of the user specific control signal determined during the communication session. In step 416, the future communication session is scheduled, 318, using the composite control signal 316.

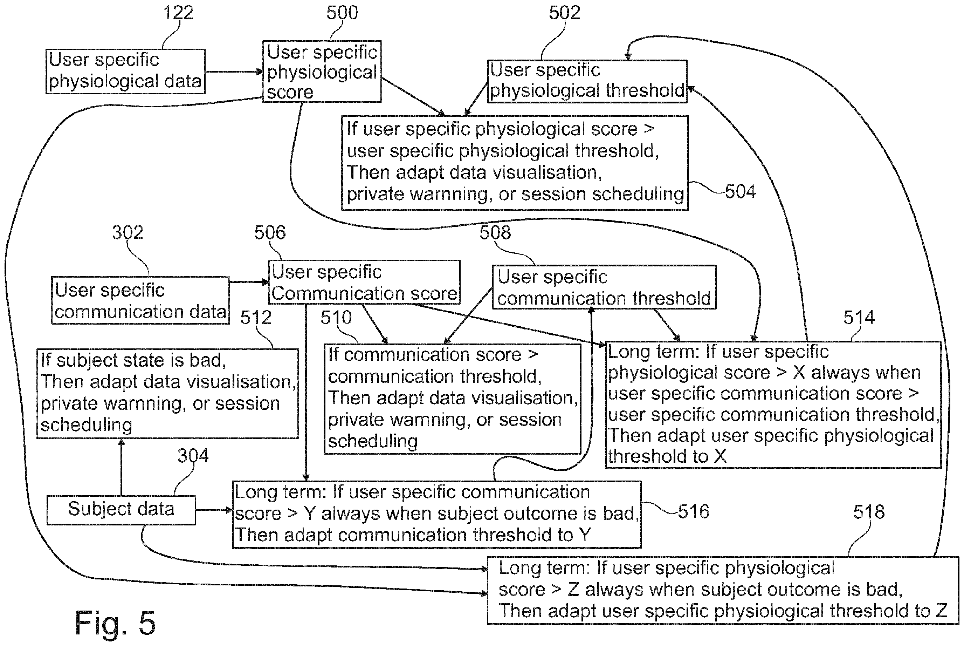

Fig. 5 illustrates how the user-specific physiological data 122, the user-specific communication data 302, and the subject data 304 may be used together to control the communication session. The user-specific physiological data 122 may be used to calculate a user-specific physiological score 500. The user-specific physiological score 500 may also be thought of as a control signal calculated from the user-specific physiological data 122. The system may also set a user-specific physiological threshold 502. In block 504, if the user-specific physiological score 500 is greater than the user-specific physiological threshold 502 then this may cause the behaviors of the communication system to be adjusted for the particular user. This may include adapting the data visualization, providing a private warning or changing future session scheduling.

The user-specific communication data 302 may be used to calculate a user-specific communication score 506. The user-specific communication score 506 may be considered to be a control signal that is calculated specifically from the user-specific communication data 302. A user-specific communication threshold 508 may be set. In block 510, if the user-specific communication score 506 is greater than the user-specific communication threshold 508 then the communication session may be adapted such as adapting the data visualization, providing a private warning, or changing session scheduling.

The subject data 304 may be monitored over time. In block 512, if the subject's data is determined to be bad or unsatisfactory for maintaining the health of the subject, then the communication session can be adapted such that the data visualization, a private warning or a session scheduling is adapted.

The user-specific physiological data 122, the user-specific communication data 302 and the subject data 304 have so far been discussed in terms of their immediate value and within a short-term time scale within the context of a closed control loop. The data 122, 302 and 304 however can be monitored long-term and the interaction between the three types of data 122, 302, 304 can be monitored and used to adapt to each other.

For example, in block 516 the user-specific communication score 506 can be monitored with respect to the subject data 304 over a longer time period. If the user-specific communication score 506 is greater than Y always when the patient outcome is bad, then the user-specific communication threshold is adapted or changed to Y. The values for blocks 304 and 506 are then used to modify the value of the user-specific communication threshold 508. The same can be performed with the user-specific physiological score 500.

In the long term, if the user-specific physiological score 500 is always greater than Z when the subject outcome is bad, then the user-specific physiological threshold is adapted or changed to the value Z. Blocks 500 and before are used to modify block 502.

The user-specific physiological data and the user-specific communication data 302 can also be used to modify the user-specific physiological threshold 502. In block 514, in the long term, if the user-specific physiological stress score is greater than *X* always when the user-specific communication score 506 is greater than the user-specific communication threshold 502 then the user-specific physiological threshold 502 is adapted or changed to *X.*

The use of the user specific physiological threshold 502 and the user specific communication threshold 508 by the medical system could enable adjustment and adaption of the communication and education platform in order to accommodate the various fatigue/stress levels (as indicated by the user specific physiological data 122) of the communicating persons (users of the multiple communication devices (104):
- The system could present the fatigue/stress levels (user specific physiological score 500) of the persons communicating, so that everyone present can take this information into account, adapting one's behavior when one's own stress levels are high, and/or accommodating the other stakeholders when their fatigue levels are high. This would also help to maintain a pleasant atmosphere among stakeholders. However, some people might feel that presenting their personal stress or fatigue levels invades their privacy. In this case, it could be a solution to only present average (or maximum) stress/fatigue levels of all present, thus protecting the privacy of the communicating partners to some way extent.
- The system could calculate and advise a preferable duration of the session so as not to overstress the participants with the highest stress levels or exhaust those with the highest fatigue levels. The preferable duration can be looked up in a population-based table matching stress levels to optimum session durations. Alternatively, for each individual the system can learn over time what his or her preferable duration is, as judged from when stress/fatigue levels start to increase during a session. The preferable duration for a session could then be determined as that of the stakeholder with the lowest preferable duration, or as a weighted averaged of the preferred durations of all stakeholders present. Also, the state of the patient (if present) and the recent behavior of the stakeholders can be taken into account.
- The medical system could also learn which exact form of stress feedback presentation is most effective for any stakeholder, by monitoring how the mental state of a certain stakeholder improves after a feedback presentation, and assessing over time which type of presentation evokes the largest reduction of fatigue or stress.
- The stress level of a stakeholder might determine how the medical information on the screen is presented, for instance the amount of information. E.g., when the stakeholder is stressed, the system might decide to present only the most important information, especially if the system has learned over time that this way of presenting is least stressful/tiring for this stakeholder. Moreover, the information could be presented in a way where more generic information is firstly showed to the user and when stress or fatigue levels are low go deeper in the granularity of the information presented. In contrast, when fatigue or stress levels are higher, crucial information would be shown to not overload the user.
- The system could adapt to schedule breaks in between sessions personalized to each user. When an increasing trend is observed in the stress or fatigue levels, a break to do some relaxation, stretching or breathing exercises could be triggered. Initially this scheduling could be general for all users (e.g. break every 45 mins.). With time the system could adapt and trigger such breaks in a personalized way. Measuring how the stress or fatigue decreases after such breaks could help in customizing the breaks scheduling in an optimized way per user.
- The system could present small lapses of humor/comedy between sessions so as to relieve the instantaneous stress of the stakeholders. This might be especially useful if the system notices that stress has increased considerably in the preceding session. Also, other forms of content that might have a relaxing impact could be presented in between sessions, like breathing exercises or restful music.
- All the above adaptations could depend in part on the state of the patient (if present). If the situation is critical, higher levels of stress are expected, and this might not be a good time to suggest a breathing exercise.

Various examples may also take into account the behavior of the communicating stakeholders (via the user specific communication score 506): is their communication fluent (are repeat questions asked?, do people interrupt each other?), are they paying attention (is their gaze directed toward the others or the images?). From this the user specific communication score 302 could be derived for this particular session.
- The communication scores accrued over time can be analyzed in relation to the fatigue/stress scores (the user specific physiological scores 500) of the participants. This allows the system to derive the individual threshold fatigue/stress level (the user specific physiological threshold 502) for each person, above which communication could possibly suffer. These personal threshold fatigue/stress levels will allow to further personalize the system described above.
- The user specific communication score 506 can also be analyzed in relation to further treatment and recovery data of the patients (subject data 304) involved. This may enable the system to determine which fatigue/stress levels 502 and communication scores 506 are so low that they negatively impact care (as derived from prolonged treatment or delayed recovery). Again, these values allow the system to decide to intervene if communication is of dangerously low quality.
- The user specific communication score 506 can take into account factors such as whether the speakers are using their native language or a second language as this may affect the efficiency of their communication. Repeated questions may not be due to stress or fatigue, but rather due to misunderstanding of language used.
- The user specific communication score 506 can be calculated with the previously mentioned methods. Audio signal processing could be used to monitor tone inflection, breathe patterns, speech speed. Automatic Speech Recognition and Natural Language processing could be used combined to monitor repetition in questions, track convergence in a discussion and measure how fluent a conversation is between stakeholders.

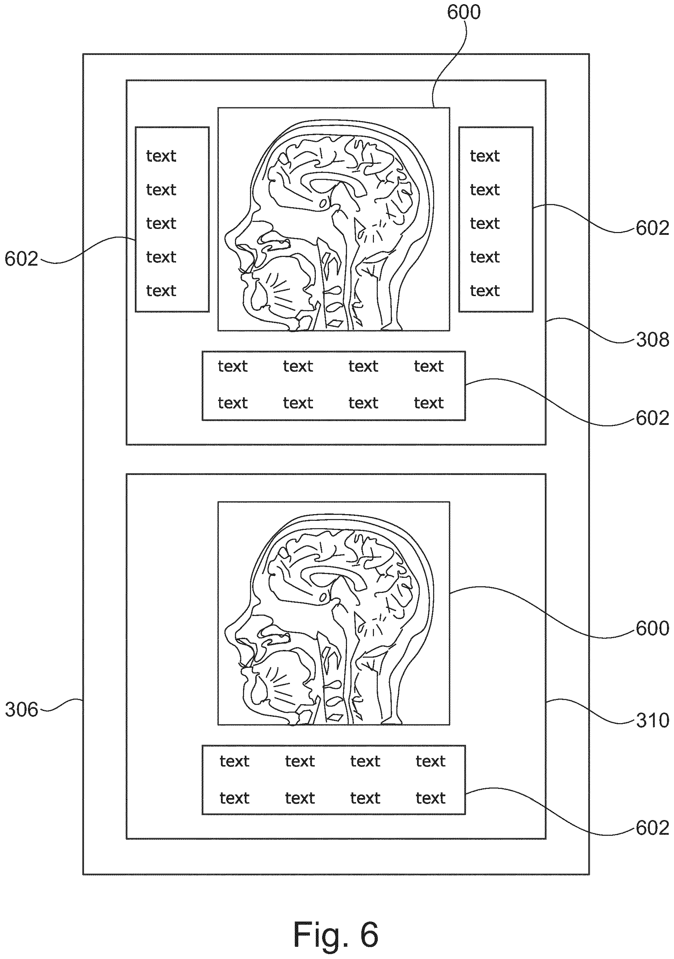

Fig. 6 illustrates a group of visual data 306. Image 308 corresponds to the visual data displayed for the first category 308 and block 310 represents the visual data displayed for the second category 310. The first category 308 contains a medical image 600 and three blocks of text 602. The second category 310 contains the medical image 600 and one block of text 602. It can be seen that the first category 308 contains much more visual data than the second category 310. If an individual is stressed, as may be indicated by the user-specific physiological score, it may be beneficial to use the second category 310 as it contains less information to understand. The user-specific control signal 124 can then therefore be used to adjust the amount of visual data presented and adapted to a particular user.

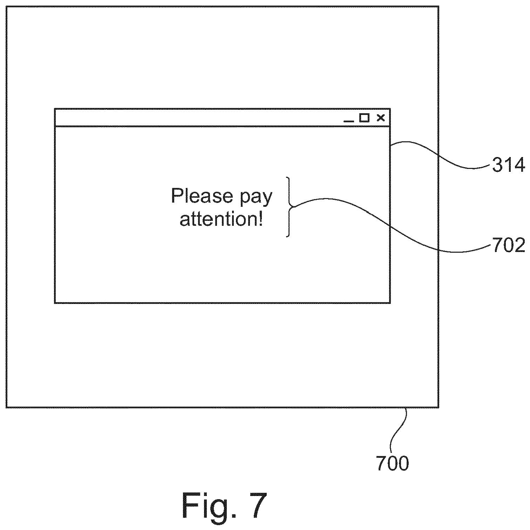

Fig. 7 illustrates an example of a user interface 700 on one of the communication devices 104. A private warning 314 has been displayed. There is a user-specific message 702 which is provided to the particular user.

It is understood that one or more of the aforementioned examples or embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A `computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

`Computer memory' or 'memory' is an example of a computer-readable storage medium. Computer memory is any memory which is directly accessible to a computational system. `Computer storage' or 'storage' is a further example of a computer-readable storage medium. Computer storage is any non-volatile computer-readable storage medium. In some embodiments computer storage may also be computer memory or vice versa.

A `computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A `user interface' as used herein is an interface which allows a user or operator to interact with a computer or computer system. A `user interface' may also be referred to as a `human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A `hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or `display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: medical system
- 102: computer
- 104: communication device
- 106: medical image source
- 108: computational system
- 110: network interface (communication system)
- 112: user interface
- 114: memory
- 120: machine executable instructions
- 122: user specific physiological data
- 124: user specific control signal
- 200: receive user specific physiological data from the multiple communication devices using the communication system
- 202: calculate a user specific control signal for the multiple telecommunication devices
- 204: control the communication session using the user specific control signal
- 300: medical system
- 302: user specific communication data
- 304: subject data
- 306: visual data
- 308: first category of visual data
- 310: second category of visual data
- 312: user specific rendering
- 314: private or public warning signal
- 316: composite control signal
- 318: schedule for future communication session
- 400: receive user specific communication data from the multiple communication devices
- 402: receive subject data descriptive of a clinical subject
- 404: use the user specific control signal to choose at least one category of the multiple categories of the visual data
- 406: construct a user specific rendering of the at least one group of visual data using the at least one category selected using the user specific control signal
- 408: transmit the user specific rendering to the multiple communication devices
- 410: provide a public warning signal or private warning signal if the user specific control signal is within a predetermined range.
- 412: session finished?
- 414: calculate a composite control signal using the user specific score received from the multiple telecommunication devices
- 416: schedule a future communication session using the composite control signal
- 500: user specific physiological score
- 502: user specific physiological threshold
- 504: If user specific physiological score is greater than the user specific physiological threshold, then adapt the data visualization, private warning, or session scheduling
- 506: user specific communication score
- 508: user specific communication threshold
- 510: if communication score is greater than communication threshold, then adapt data visualization, private warning, or session scheduling
- 512: if subject state is bad or unsatisfactory, then adapt data visualization, private warning, or session scheduling
- 514: Long term: if user specific physiological score is greater than X always when the user specific communication score is greater than the user specific communication threshold, then adapt the user specific physiological threshold to X
- 516: Long term: if the user specific communication score is greater than Y when ever the subject outcome is bad or unsatisfactory, then adapt user specific communication threshold to Y
- 518: Long term: if the user specific physiological score is greater than Z whenever the subject outcome is bad or unsatisfactory, then adapt the user specific physiological threshold to Y
- 600: medical image
- 602: block of text
- 700: user interface
- 702: message

## Claims

1. A medical system (100, 300) comprising:
- a communication system (110) configured for establishing a communication session between multiple communication devices (104);
- a memory (114) storing machine executable instructions (120);
- a computational system, wherein execution of the machine executable instructions causes the computational system to repeatedly:
- receive (200) user specific physiological data (122) from the multiple communication devices using the communication system;
- calculate (202) a user specific control signal (124) for the multiple telecommunication devices at least partially using the user specific physiological data; and
- control (204) the communication session using the user specific control signal.

2. The medical system of claim 1, wherein the memory further stores at least one group of visual data (306), wherein the at least one group of visual data is divided into multiple categories (308, 310), wherein execution of the machine executable instructions further causes the computational system to repeatedly:
- use (404) the user specific control signal to choose at least one category of the multiple categories;
- construct (406) a user specific rendering (312) of the at least one group of visual data using the at least one category selected using the user specific control signal; and
- transmit (408) the user specific rendering to the multiple communication devices.

3. The medical system of claim 2, wherein the medical system further comprises any one of the following:
- a medical imaging system (106), wherein the at least one group of visual data comprises acquired medical images and/or a control user interface rendering;
- a medical image database (106), wherein the at least one group of visual data at least partially comprises data retrieved from the medical image database; and
- combinations thereof.

4. The medical system of claim 1, 2, or 3, wherein execution of the machine executable instructions further causes the computational system to provide (410) a public warning signal or private warning signal (314) if the user specific control signal is within a predetermined range.

5. The medical system of any one of the preceding claims, wherein execution of the machine executable instructions further causes the computational system to:
- calculate (414) a composite control signal (316) using the user specific score received from the multiple telecommunication devices; and
- schedule (416) a future communication session (318) using the composite control signal.

6. The medical system of any one of the preceding claims, wherein the user specific physiological data comprises sensor data collected by the multiple communication devices.

7. The medical system of any one of the preceding claims, wherein execution of the machine executable instructions causes the computational system to repeatedly receive (400) user specific communication data (302) from the multiple communication devices, and wherein the user specific control signal is at least partially calculated using the user specific communication data.

8. The medical system of claim 7, wherein the user specific control signal is calculated using a correlation between the user specific communication data and the user specific physiological data.

9. The medical system of claim 7 or 8, wherein the communication data comprises a user specific audio stream collected by the multiple telecommunication communication devices, wherein the memory further comprises a speech recognition module configured to output one or more speech metric in response to receiving the user specific audio stream, wherein execution of the machine executable instructions further causes the computational system to receive the one or more speech metric in response to inputting the user specific audio stream into the speech recognition module, wherein execution of the machine executable instructions further causes the computational system to at least partially calculate the user specific control signal using the one or more speech metric.

10. The medical system of claim 7, 8, of 9, wherein the user specific communication data comprises a video stream comprising user specific images collected by the multiple communication devices, wherein the wherein the memory further comprises an image recognition module configured to output one or more image metric in response to receiving the video stream, wherein execution of the machine executable instructions further causes the computational system to receive the one or more image metric in response to inputting the image stream into the image recognition module, wherein execution of the machine executable instructions further causes the computational system to at least partially calculate the user specific control signal using the one or more image metric.

11. The medical system of any one of claims 7 through 10, wherein the user specific communication data further comprise text metadata, wherein the user specific control signal is at least partially calculated using the text metadata, wherein the text metadata comprises any one of the following collected by the multiple communication devices:
- a user specific reaction time to a received message;
- a user specific typing speed;
- user specific questionnaire responses; and
- user specific small talk response data received in response to chatbot conversation data.

12. The medical system of any one of claims 7 through 11, wherein execution of the machine executable instructions further causes the computational system to receive (402) subject data (304) descriptive of a clinical subject, wherein the user specific control signal is at least partially calculated using the subject data.

13. The medical system of claim 12, wherein execution of the machine executable instructions further causes the computational system to adjust the user specific control signal using a correlation between the user specific communication data and the subject data.

14. A method of operating a medical system (100, 300), wherein the medical system comprises a communication system (110) configured for establishing a communication session between multiple communication devices (104), wherein the method comprises repeatedly:
- receiving (200) user specific physiological data (122) from the multiple communication devices using the communication system;
- calculating (202) a user specific control signal (124) for the multiple telecommunication devices at least partially using the user specific physiological data; and
- controlling (204) the communication session using the user specific control signal.

15. A computer program comprising machine executable instructions (120) for execution by a computational system (108) controlling a communication system (110), wherein execution of the machine executable instructions causes the computational system to repeatedly:
- receive (200) user specific physiological data (122) from the multiple communication devices using the communication system;
- calculate (202) a user specific control signal (124) for the multiple telecommunication devices at least partially using the user specific physiological data; and
- control (204) the communication session using the user specific control signal.
